# EUROPEAN PATENT SPECIFICATION

(11) **EP 3 585 360 B1**
(45) Date of publication and mention of the grant of the patent: **22.01.2025**
(21) Application number: 18708189.8
(22) Date of filing: 23.02.2018
(51) Int. Cl.: A61K 9/08, A61K 9/00, A61K 38/17, A61K 39/395

(54) **STABILIZED ANTIBODY SOLUTIONS**
STABILISIERTE ANTIKÖRPER-ENTHALTENDE LÖSUNGEN
SOLUTIONS STABILISÉES CONTENANT DES ANTICORPS

(30) Priority: 24.02.2017 GB 201703062
(43) Date of publication of application: 01.01.2020
(73) Proprietor: Arecor Limited, Little Chesterford Saffron Walden CB10 1XL (GB)
(72) Inventor: JEZEK, Jan, Saffron Walden CB10 1XL (GB); GERRING, David, Saffron Walden CB10 1XL (GB)
(74) Representative: Sagittarius IP
(86) International application number: PCT/GB2018/050481
(87) International publication number: WO 2018/154320

(56) References cited:
- WO-A1-2018/011404
- US-A1- 2003 138 417
- US-A1- 2008 112 953
- US-A1- 2013 209 465
- US-B2- 8 241 632
- WANG WEI ED - BLANCO-PRIETO MARIA J ET AL: "Instability, stabilization, and formulation of liquid protein pharmaceuticals", INTERNATIONAL JOURNAL OF PHARMACEUTICS, ELSEVIER, NL, vol. 185, no. 2, 20 August 1999 (1999-08-20), pages 129 - 188, XP002323952, ISSN: 0378-5173, DOI: 10.1016/S0378-5173(99)00152-0
- WANG W ET AL: "ANTIBODY STRUCTURE, INSTABILITY, AND FORMULATION", JOURNAL OF PHARMACEUTICAL SCIENCES, AMERICAN CHEMICAL SOCIETY AND AMERICAN PHARMACEUTICAL ASSOCIATION, US, vol. 96, no. 1, 1 January 2007 (2007-01-01), pages 1 - 26, XP009084505, ISSN: 0022-3549, DOI: 10.1002/JPS.20727
- DAUGHERTY A L ET AL: "Formulation and delivery issues for monoclonal antibody therapeutics", ADVANCED DRUG DELIVERY REVIEWS, ELSEVIER, AMSTERDAM , NL, vol. 58, no. 5-6, 7 August 2006 (2006-08-07), pages 686 - 706, XP024892149, ISSN: 0169-409X, [retrieved on 20060807], DOI: 10.1016/J.ADDR.2006.03.011

## Description

### BACKGROUND OF THE INVENTION

When formulated as aqueous solutions, antibody proteins are susceptible to structural degradation during storage. The processes involved in protein degradation can be divided into physical (e.g. loss of quaternary, tertiary or secondary structure, aggregation, particle formation) and chemical (i.e. processes involving a covalent change such as deamidation, aspartate isomerization, oxidation, hydrolytic clipping etc.). Each of the degradants (e.g. soluble aggregated species, insoluble aggregated species and chemically modified variants) can impact the biological activity, toxicity or immunogenicity of the antibody protein.

Therefore, the level of all degradants has to be kept within the tight specifications that are set for each antibody protein product. The rates of the degradation processes depend on temperature and antibody proteins are generally more stable at lower temperatures. Consequently, commercial antibody products must typically be stored refrigerated. However, with increasing trend toward subcutaneous products that can be self-administered by the patient, there is a strong need to develop antibody protein products that can be used outside the cold chain, at least for a period of time, such as 2 weeks, such as 4 weeks, such as 12 weeks or more. The ability to store the product outside the cold chain often results in considerable improvement in convenience for the patient during the in-use period. Allowed excursions outside the cold chain can also significantly improve shipment logistics.

The present invention addresses the problem of instability of antibody proteins, in particular the problem of antibody protein degradation.

WO2006/0096488A2 (Pharmacia & Upjohn Company LLC) describes compositions of human IgG antibodies comprising a chelating agent, said to exhibit improved chemical and/or physical stability.

WO2013/114122A2 (Arecor Limited) describes an aqueous solution comprising an antibody protein at a concentration of at least about 10 mg/mL and an oligomer of ethyleneimine, wherein the number of repeating units of ethyleneimine (n) in the oligomer is in the range of n = 2-12.

WO2010/062896A1 (Abbott Laboratories) describes compositions and methods for inhibiting fractionation of immunoglobulins comprising a lambda light chain based on the observation that iron, in the presence of histidine, results in increased fragmentation of a recombinant fully human IgG molecule containing a lambda light chain due to cleavage in the hinge region.

US2013/0209465A1 (Arecor Limited) describes an aqueous solution comprising an antibody protein at a concentration of at least about 10 mg/mL and a stabilising amount of polyethyleneimine.

US2008/112953A1 (Amgen Inc.) describes a formulation including an acetic acid buffer, a glutamic acid buffer, or a succinic acid buffer with a pH from about 4.5-7.0, at least one excipient comprising a sugar or a polyol and an effective amount of a therapeutic antibody.

US8241632B2 (Amgen Inc.) describes a formulation including a buffer having a pH less than 6.0, a divalent cation between about 5-200 mM, an excipients comprising a sugar or polyol and an effective amount of a therapeutic polypeptide.

US2003/138417A1 (Kaisheva et al.) describes a stable liquid pharmaceutical formulation comprising a high concentration e.g. 50 mg/mL or more, of antibody in about 20-60 mM succinate buffer or 30-70 mM histidine buffer, having pH from about pH 5.5 to about pH 6.5, about 0.01-0.1% polysorbate, and a tonicity modifier that contributes to the isotonicity of the formulation.

WO2018/011404A1 (Philogen S.P.A) describes stable formulations that can be prepared to high concentration of active agent using low amounts of detergents.

Wang et al. 1999 is a review of the basic behaviour of proteins, their instabilities, and stabilization in aqueous state in relation to the development of liquid protein pharmaceuticals.

Wang et al. 2007 is a review of the structure and function of antibodies and the mechanisms of physical and chemical instabilities.

Daugherty et al. 2006 is a review focused on issues related to identifying and verifying stable antibody-based formulations.

### SUMMARY OF THE INVENTION

The present invention addresses the problem of instability of antibody proteins. Specifically, the invention set out in the appended set of claims relates to an aqueous solution comprising an antibody protein which is a monoclonal antibody wherein the antibody protein is an IgG immunoglobulin present at a concentration of 10 to 200 mg/ml and a stabilizing mixture of (i) a chelating agent which is EDTA present at a concentration of 0.1 to 10 mM; (ii) a C3 polyol which is selected from glycerol and 1,2-propanediol present at a concentration of 100 to 500 mM; (iii) a non-ionic surfactant present at a concentration of 10 to 2000 µg/ml and (iv) a buffer.

### DETAILED DESCRIPTION OF THE INVENTION

The present invention relates to the discovery that an aqueous solution of antibody protein can be stabilized by a mixture of a chelating agent which is EDTA, and a C3 polyol which is selected from glycerol and 1,2-propanediol.

The term "aqueous solution", as used herein, refers to a solution in water, preferably distilled water, deionized water, water for injection, sterile water for injection or bacteriostatic water for injection. The aqueous solutions of the invention include dissolved antibody protein which is a monoclonal antibody, wherein the antibody protein is an IgG immunoglobulin, a chelating agent which is EDTA, a C3 polyol which is selected from glycerol and 1,2-propanediol, a non-ionic surfactant, and a buffer, and optionally, one or more additives and/or excipients. The aqueous solutions can also include one or more components, such as additives or excipients, which are partially dissolved or undissolved. The presence of such component or components will result in a multi-phase composition, such as a suspension or an emulsion. Preferably, the aqueous solution of the invention is a homogeneous solution, as determined by eye or by light-scattering.

The term "antibody protein", as used herein refers to an antibody, an antibody fragment, an antibody conjugated to an active moiety, a fusion protein comprising one or more antibody fragments, such as an immunoglobulin Fc domain, or a derivative of any of the aforementioned. Examples of derivatives include conjugated derivatives e.g. an antibody or antibody fragment conjugated to another moiety. Such moieties include chemically inert polymers such as PEG. The antibody protein of the invention set out in the appended set of claims is a monoclonal antibody wherein the antibody protein is an IgG immunoglobulin. The monoclonal antibodies can be, for example, mammalian (e.g. murine) or avian, chimeric, for example, human/mouse or human/primate chimeras, humanized antibodies or fully human antibodies. Suitable antibodies include an IgG₁, IgG₂, IgG₃ or IgG₄ immunoglobulin. Suitable antibodies also include single chain antibodies.

In certain embodiments, the monoclonal antibody, which is an IgG immunoglobulin, is fused or conjugated to an active molecule, such as a toxin or a chelating agent capable of binding a radioactive metal ion, such as ⁹⁹Tc, ¹¹¹Ir, ¹³¹I or ⁹⁰Y. In such embodiments, the monoclonal antibody, which is an IgG immunoglobulin, typically functions as a targeting agent, for example, directing the active molecule to cells which display a certain cell surface protein.

Specific antibodies which can be formulated as described herein include, but are not limited to, infliximab (chimeric antibody, anti-TNFα), basiliximab (chimeric antibody, anti-IL-2), abciximab (chimeric antibody, anti- GpIIb/IIIa), daclizumab (humanized antibody, anti-IL-2), gemtuzumab (humanized antibody, anti-CD33), alemtuzumab (humanized antibody, anti-CD52), edrecolomab (murine Ig2a, anti-EpCAM), rituximab (chimeric antibody, anti-CD20), palivizumab (humanized antibody, anti-respiratory syncytial virus), trastuzumab (humanized antibody, anti- HER2/neu(erbB2) receptor), bevacizumab (humanized antibody, anti-VEGF), cetuximab (chimeric antibody, anti-EGFR), eculizumab (humanized antibody, anti-complement system protein C5), efalizumab (humanized antibody, anti-CD 1Ia), ibritumomab (murine antibody, anti-CD20), muromonab-CD3 (murine antibody, anti- T cell CD3 receptor), natalizumab (humanized antibody, anti-α4 integrin), nimotuzumab (humanized IgGl, anti-EGF receptor), omalizumab (humanized antibody, anti-IgE), panitumumab (human antibody, anti-EGFR), ranibizumab (humanized antibody, anti-VEGF), 1-131 tositumomab (humanized antibody, anti-CD20), ofatumumab (human antibody, anti-CD-20), certolizumab (humanized antibody, anti-TNF-α), golimumab (human antibody, anti-TNFα) and denosumab (human antibody, anti-RANK ligand). Preferred antibodies include trastuzumab, rituximab, bevacizumab, cetuximab and ipilimumab. In one embodiment, the antibody is bevacizumab. In one embodiment, the antibody is not an anti-TNF-α antibody.

Other chimeric antibodies which can be formulated as described herein include bavituximab (anti-phosphatidylserine), brentuximab (anti-CD30), siltuximab (anti-IL-6), clenoliximab (anti-CD4), galiximab (anti-CD80), gomiliximab (anti-CD23), keliximab (anti-CD4), lumiliximab (anti-CD23), priliximab (anti-CD4), teneliximab (anti-CD40), vapaliximab (anti-VAP1), ecromeximab (anti-GD3), and pagibaximab (anti-staphylococcal lipoteichoic acid).

Other humanized antibodies which can formulated as described herein include epratuzumab (anti-CD22), afutuzumab (anti-CD20), bivatuzumab mertansine (anti-CD44), cantuzumab mertansine (anti-mucin), citatuzumab bogatox (anti-TACSTD1), dacetuzumab (anti-CD40), elotuzumab (anti-CD319), etaracizumab (anti-αᵥβ₃-integrin), farletuzumab (anti-FRα), inotuzumab ozogamicin (anti-CD22), labetuzumab (anti-carcinoembryonic antigen), lintuzumab (anti-CD33), milatuzumab (anti-CD74), nimotuzumab (anti-EGFR), oportuzumab monatox (anti-EpCAM), pertuzumab (anti-HER2), sibrotuzumab (anti-FAP), tacatuzumab tetraxetan (anti-alpha-fetoprotein), tigatuzumab (anti-TRAIL-2), tucotuzumab celmoleukin (anti-EpCAM), veltuzumab (anti-CD20), aselizumab (anti-CD62L), apolizumab (anti-HLA-DRB), benralizumab (anti-CD125), cedelizumab (anti-CD4), epratuzumab (anti-CD22), erlizumab (anti-CD18), fontolizumab (anti-interferon-γ), mepolizumab (anti-IL5), ocrelizumab (anti-CD20), pascolizumab (anti-IL4), pexelizumab (anti-complement component 5), PRO-140 (anti-CCR5), reslizumab (anti-IL5), rontalizumab (anti interferon-α), rovelizumab (anti-CD11, CD18), siplizumab (anti-CD2), talizumab (anti-IgE), teplizumab (anti-CD3), tocilizumab (anti-IL6R), vedolizumab (anti-α₄β₇-integrin), visilizumab (anti-CD3), ibalizumab (anti-CD4), tefibazumab (anti-clumping factor A), tadocizumab (anti-α_{11b}β₃-integrin), bapineuzumab (anti-amyloid-β), solanezumab (anti-amyloid-β), tanezumab (anti-NGF), urtoxazumab (anti-E. coli Shiga-like toxin II B subunit), felvizumab (anti-respiratory syncytial virus), motavizumab (anti- respiratory syncytial virus glycoprotein F) and lebrikizumab (anti-IL13).

Additional human antibodies which can be formulated as described herein include atorolimumab (anti-Rh factor), fresolimumab (anti-TGFβ-1, -2, and -3), lerdelimumab (anti-TGFβ-2), metelimumab (anti-TGFβ-1), morolimumab (anti-Rh factor), ipilimumab (anti-CTLA-4), tremelimumab (anti-CTLA-4), bertilimumab (anti-CCL11), zanolimumab (anti-CD4), briakinumab (anti-IL12, -23), canakinumab (anti-IL1β), ustekinumab (anti-IL12, -23), adecatumumab (anti-EpCAM), belimumab (anti-B cell activating factor), cixutumumab anti-IGF-1 receptor), conatumumab (anti-TRAIL-R2), figitumumab (anti-IGF-1 receptor), iratumumab (anti-CD30), lexatumumab (anti-TRAIL-R2), lucatumumab (anti-CD40), mapatumumab (anti-TRAIL-R4), necitumumab (anti-EGFR), olaratumab (anti-PDGF-Rα), pritumumab (anti-vimentin), robatumumab (anti-IGF-1 receptor), votumumab (anti-tumor antigen CTAA16.88), zalutumumab (anti-EGFR), stamulumab (anti-myostatin), efungumab (anti-fungal HSP90), exbivirumab (anti-hepatitis B surface antigen), foravirumab (anti- rabies glycoprotein), libivirumab (anti-hepatitis B surface antigen), rafivirumab (anti- rabies glycoprotein), regavirumab (anti-cytomegalovirus glycoprotein B), sevirumab (anti-cytomegalovirus), tuvirumab (anti-hepatitis B virus), panobacumab (anti-pseudomonas aeruginosa serotype IATS 011), raxibacumab (anti-anthrax toxin), ramucirumab (anti-VEGF-R2), and gantenerumab (anti-amyloid-β).

In certain embodiments , conjugated derivatives comprising monoclonal antibodies, which are IgG immunoglobulins, and a chemically inert polymer such as PEG can also be formulated according to the invention. Such derivatives include certolizumab pegol.

The antibody protein can be isolated from natural sources or be a recombinant protein.

In certain embodiments, the antibody protein is substantially pure, that is, the composition comprises a single antibody protein and no substantial amount of any additional protein. In preferred embodiments, the antibody protein comprises at least 99%, preferably at least 99.5% and more preferably at least about 99.9% of the total protein content of the composition. In preferred embodiments the antibody protein is sufficiently pure for use as in a pharmaceutical composition.

The antibody protein is preferably a therapeutic antibody protein. Such an antibody protein has a desirable therapeutic or prophylactic activity and is indicated for the treatment, inhibition or prevention of a disease or medical disorder.

In one embodiment, the antibody protein is a monoclonal antibody, wherein the antibody protein is an IgG immunoglobulin, selected from trastuzumab, rituximab, bevacizumab, cetuximab and ipilimumab.

The antibody protein is present at a concentration of 10 mg/mL to 200 mg/mL.

The aqueous solution of the present invention comprises a chelating agent which is a multi-anion, which is EDTA, as a stabilizing agent. By multi-anion, what is meant is a species which has at least two anionic centres per molecule, at the particular pH of the solution. By chelating agent what is meant is an agent capable of complexing with metal ions such as calcium, magnesium, iron and/or zinc ions. EDTA anion is preferably introduced into the aqueous solution in the form of a salt of ethylenediaminetetraacetatic acid, such as disodium or tetrasodium salt. Alternatively, it can be introduced in the form of ethylenediaminetetraacetatic acid with subsequent adjustment of pH to the required level.. EDTA may be employed as a suitable salt form (e.g. as a sodium salt), or as an acid form which forms a multi-anion in solution. EDTA is present at a concentration of 0.1 mM to 10 mM.

The aqueous solution of the invention also comprises a C3 polyol as a stabilizing agent which is selected from 1,2-propanediol (also known as propane-1,2-diol or propylene glycol) and glycerol (also known as 1,2,3-propanetriol, glycerin or glycerine). In one embodiment, the C3 polyol is 1,2-propanediol. In another embodiment, the C3 polyol is glycerol. In a further embodiment, the C3 polyol is a mixture of 1,2-propanediol and glycerol. The C3 polyol is present at a concentration of 100 mM to 500 mM, such as about 150 mM to about 400 mM, or about 150 mM to about 300 mM. If more than one C3 polyol is present in the aqueous solution, then the concentration refers to the total concentration of C3 polyols.

Typically, the pH of the aqueous solution of the present invention is between pH 4.0 and pH 8.0, such as between pH 5.0 and pH 7.0 or between pH 5.0 and pH 6.5.

The aqueous solution of the invention further comprises a buffer in order to stabilise the pH of the formulation, which can also be selected to enhance antibody protein stability. Suitably the buffer is selected from the group consisting of histidine, succinate, maleate, acetate, phosphate and TRIS. In an embodiment, the buffer is phosphate buffer.

In one embodiment, a buffer is selected to have a pKₐ close to the pH of the composition; for example, histidine is suitably employed as a buffer when the pH of the composition is in the range 5.0-7.0. As another example, phosphate is suitably employed as a buffer when the pH of the composition is in the range 6.1-8.1. Alternatively, in another embodiment, the solution of the invention is further stabilised as disclosed in WO2008/084237A2, which describes a formulation comprising a protein and one or more additives, characterised in that the system is substantially free of a conventional buffer, i.e. a compound with an ionisable group having a pKₐ within 1 unit of the pH of the formulation at the intended temperature range of storage of the composition, such as 25 °C. In this embodiment, the pH of the formulation is set to a value at which the formulation has maximum measurable stability with respect to pH; the one or more additives (displaced buffers) are capable of exchanging protons with the insulin compound and have pKₐ values at least 1 unit more or less than the pH of the formulation at the intended temperature range of storage of the formulation. The additives may have ionisable groups having pKₐ between 1 to 5 pH units, preferably between 1 to 3 pH units, most preferably from 1.5 to 2.5 pH units, of the pH of the aqueous formulation at the intended temperature range of storage of the composition (e.g. 25 °C). Such additives may typically be employed at a concentration of 0.5-10 mM e.g. 2-5 mM.

Typically, the buffer is present at a concentration of about 0.5 mM to about 50 mM, such as about 1 mM to about 20 mM, e.g. about 2 mM to about 5 mM.

The aqueous solutions of the invention comprise a non-ionic surfactant such as an alkyl glycoside e.g. dodecyl maltoside; a polysorbate surfactant such as polysorbate 80 or polysorbate 20; an alkyl ether of polyethylene glycol e.g. selected from polyethylene glycol (2) dodecyl ether, polyethylene glycol (2) oleyl ether and polyethylene glycol (2) hexadecyl ether; a block copolymer of polyethylene glycol and polypropylene glycol, such as poloxamer 188, poloxamer 407, poloxamer 171 or poloxamer 185; or an alkylphenyl ether of polyethylene glycol, such as 4-(1,1,3,3-tetramethylbutyl)phenyl-polyethylene glycol. The non-ionic surfactant is present at a concentration of 10 µg/mL to 2000 µg/mL, such as about 50 µg/mL to about 1000 µg/mL, e.g. about 100 µg/mL to about 500 µg/mL.

The aqueous solution of the invention may cover a wide range of osmolarity, including hypotonic, isotonic and hypertonic aqueous solutions. Suitably, the aqueous solution of the invention is substantially isotonic. In one embodiment, the aqueous solution of the invention is isotonic. Suitably, the osmolarity of the aqueous solution is selected to minimize pain according to the route of administration e.g. upon injection. Preferred aqueous solutions have an osmolarity in the range of about 200 mOsm/L to about 500 mOsm/L. Preferably, the osmolarity is in the range of about 250 mOsm/L to about 350 mOsm/L. More preferably, the osmolarity is about 300 mOsm/L.

Tonicity of the aqueous solution may be adjusted with a tonicity modifier. Tonicity modifiers may be charged or uncharged.

Examples of charged tonicity modifiers include salts such as a combination of sodium, potassium, magnesium or calcium ions, with chloride, sulfate, carbonate, sulfite, nitrate, lactate, succinate, acetate or maleate ions (especially sodium chloride or sodium sulphate, particularly sodium chloride). Amino acids such as glycine, histidine or arginine may also be used for this purpose. In one embodiment, the charged tonicity modifier is selected from the group consisting of sodium chloride, sodium sulphate, sodium acetate, sodium lactate, glycine, histidine and arginine. Such a charged tonicity modifier is typically present at a concentration of about 25 mM to about 500 mM, such as about 50 mM to about 250 mM, e.g. about 150 mM.

Examples of uncharged tonicity modifiers include sugars, sugar alcohols and other polyols, such as sucrose, trehalose, mannitol, raffinose, lactose, dextrose, sorbitol or lactitol, or polyethylene glycols such as PEG300 or PEG400. In one embodiment, the uncharged tonicity modifier is sucrose, trehalose, mannitol, sorbitol, PEG300 or PEG400. The C3 polyol selected from glycerol and 1,2-propanediol which is a required component of the aqueous solution of the invention may function as an uncharged tonicity modifier. However, reference to an aqueous solution of the invention "further" comprising an uncharged tonicity modifier is intended to refer to an additional, further component to be added to the solution. Thus, the aqueous solution may further comprise an uncharged tonicity modifier which is other than 1,2-propanediol and glycerol. Such an uncharged tonicity modifier is typically present at a concentration of about 50 mM to about 1000 mM, such as about 100 mM to about 500 mM, e.g. about 300 mM.

The aqueous solution of the invention can optionally include a preservative, suitably selected from phenol, m-cresol, chlorocresol, benzyl alcohol, propylparaben, methylparaben, benzalkonium chloride and benzethonium chloride. When present, the preservative is at a concentration of about 0.01 mM to about 100 mM. A preservative selected from phenol, m-cresol, chlorocresol, benzyl alcohol, propylparaben, methylparaben may, for example, be present at a concentration of about 10 mM to about 100 mM, such as about 20 mM to about 80 mM e.g. about 25 mM to about 50 mM. A preservative selected from benzalkonium chloride and benzethonium chloride may, for example, be present at a concentration of about 0.01 mM to about 1 mM such as about 0.05 mM to about 0.5 mM e.g. about 0.05 mM to about 0.2 mM.

The present inventors have discovered that the stability of an antibody protein which is a monoclonal antibody, wherein the antibody protein is an IgG immunoglobulin, in an aqueous solution is improved by the addition of a mixture of (i) a chelating agent which EDTA present at a concentration of 0.1 to 10 mM; (ii) a C3 polyol which is selected from glycerol and 1,2-propanediol, present at a concentration of 100 to 500 mM; (iii) a non-ionic surfactant present at a concentration of 10 to 2000 µg/mL; and (iv) a buffer. The addition of a chelating agent which is EDTA has been observed to enhance the stability of an antibody protein which is a monoclonal antibody, wherein the antibody protein is an IgG immunoglobulin, in an aqueous solution. Surprisingly, the stabilizing effect of EDTA is further increased by the addition of a C3 polyol which is selected from glycerol and 1,2-propanediol. Without wishing to be bound by theory it is believed that the stabilising effect of the chelating agent which is EDTA is due to a combination of (i) charge interactions with positively charged patches at the surface of the protein and (ii) elimination of trace metals that may catalyse degradation processes. Without wishing to be bound by theory it is believed that the additional stabilising effect of a C3 polyol which is selected from glycerol and 1,2-propanediol, is due to optimal hydrophobic and hydrogen bond interactions at the protein surface of the small polyols leading to tighter conformation and modified interfacial tension between the protein molecules, in turn leading to lower exposure of reaction sites as well as lower probability of irreversible aggregation events.

In one embodiment, the ratio (mM/mM) of chelating agent which is EDTA to C3 polyol which is selected from glycerol and 1,2-propanediol is between about 1:5 and about 1:500 e.g. between about 1:20 and about 1:200.

In one embodiment, the ratio (wt/wt) of antibody protein which is a monoclonal antibody, wherein the antibody protein is an IgG immunoglobulin, to chelating agent which is EDTA is between about 1:1 and about 500:1 e.g. between about 10:1 and about 200:1. In another embodiment, the ratio (wt/wt) of antibody protein which is a monoclonal antibody, wherein the antibody protein is an IgG immunoglobulin, to chelating agent which is EDTA is between about 10:1 and about 1000:1 e.g. between about 50:1 and about 200:1.

In one embodiment, the ratio (wt/wt) of antibody protein which is a monoclonal antibody, wherein the antibody protein is an IgG immunoglobulin, to C3 polyol which is selected from glycerol and 1,2-propanediol is between about 1:5 and about 200:1 e.g. between about 1:1 and about 50:1. In another embodiment, the ratio (wt/wt) of antibody protein which is a monoclonal antibody, wherein the antibody protein is an IgG immunoglobulin, to C3 polyol which is selected from glycerol and 1,2-propanediol is between about 1:2 and about 200:1 e.g. between about 2:1 and about 50:1.

The addition of a mixture of chelator which is EDTA, and a C3 polyol which is selected from glycerol and 1,2-propanediol to an aqueous solution of antibody protein which is a monoclonal antibody, wherein the antibody protein is an IgG immunoglobulin, is expected to enhance the stability of the antibody protein, e.g. as shown in Example 1. The mixture of a chelating agent which is EDTA and a C3 polyol which is selected from glycerol and 1,2-propanediol is thus referred to as a stabilizing mixture.

The "stability" of an antibody protein or a "stabilizing mixture" typically refers to a reduction of antibody protein degradation during storage. In one embodiment, "stability"/"stabilizing" refers to physical stability e.g. loss of quaternary, tertiary or secondary structure, aggregation or particle formation. In another embodiment, "stability"/"stabilizing" refers to chemical stability e.g. processes involving a covalent change such as deamidation, aspartate isomerization, oxidation or hydrolytic clipping.

It is expected that the addition of a mixture of a chelating agent which is EDTA, and a C3 polyol which is selected from glycerol and 1,2-propanediol, to an aqueous solution comprising an antibody protein which is a monoclonal antibody, wherein the antibody protein is an IgG immunoglobulin can enhance the stability of the antibody protein and in particular reduce the rate of antibody protein aggregation, compared with the same solution lacking the chelating agent which is EDTA, and a C3 polyol which is selected from glycerol and 1,2-propanediol, following storage under the same conditions for the same length of time.

The present invention thus provides the use of a mixture of (i) a chelating agent which is EDTA present at a concentration of 0.1 to 10 mM; (ii) a C3 polyol selected from glycerol and 1,2-propanediol present at a concentration of 100 to 500 mM; (iii) a non-ionic surfactant present at a concentration of 10 to 2000 µg/mL; and (iv) a buffer, for stabilizing an antibody protein which is a monoclonal antibody, wherein the antibody protein is an IgG immunoglobulin present at a concentration of 10 to 200 mg/mL in an aqueous solution to storage. All embodiments described hereinabove with reference to the aqueous solution of the invention apply equally to the use of the invention.

Also provided is the use of a mixture of a chelating agent which is EDTA present at a concentration of 0.1 to 10 mM, (ii) a C3 polyol selected from glycerol and 1,2-propanediol present at a concentration of 100 to 500 mM; (iii) a non-ionic surfactant present at a concentration of 10 to 2000 µg/mL; and (iv) a buffer for inhibiting the formation of high molecular weight species of an antibody protein which is a monoclonal antibody, wherein the antibody protein is an IgG immunoglobulin present at a concentration of 10 to 200 mg/mL in aqueous solution during storage.

Also provided is the use of a mixture of a chelating agent which is EDTA present at a concentration of 0.1 to 10 mM, (ii) a C3 polyol selected from glycerol and 1,2-propanediol present at a concentration of 100 to 500 mM; (iii) a non-ionic surfactant present at a concentration of 10 to 2000 µg/mL; and (iv) a buffer for inhibiting the formation of visible particles in an aqueous solution of an antibody protein which is a monoclonal antibody, wherein the antibody protein is an IgG immunoglobulin present at a concentration of 10 to 200 mg/mL during storage.

Also provided is the use of a mixture of a chelating agent which is EDTA present at a concentration of 0.1 to 10 mM, (ii) a C3 polyol selected from glycerol and 1,2-propanediol present at a concentration of 100 to 500 mM; (iii) a non-ionic surfactant present at a concentration of 10 to 2000 µg/mL; and (iv) a buffer for inhibiting formation of related species of an antibody protein which is a monoclonal antibody wherein the antibody protein is an IgG immunoglobulin present at a concentration of 10 to 200 mg/mL in aqueous solution during storage.

Also provided is the use of a mixture of a chelating agent which is EDTA present at a concentration of 0.1 to 10 mM, (ii) a C3 polyol selected from glycerol and 1,2-propanediol present at a concentration of 100 to 500 mM; (iii) a non-ionic surfactant present at a concentration of 10 to 2000 µg/mL; and (iv) a buffer for inhibiting deamidation of an antibody protein which is a monoclonal antibody wherein the antibody protein is an IgG immunoglobulin present at a concentration of 10 to 200 mg/mL in aqueous solution during storage.

Also provided is the use of a mixture of a chelating agent which is EDTA present at a concentration of 0.1 to 10 mM, (ii) a C3 polyol selected from glycerol and 1,2-propanediol present at a concentration of 100 to 500 mM; (iii) a non-ionic surfactant present at a concentration of 10 to 2000 µg/mL; and (iv) a buffer for inhibiting formation of low molecular weight degradation products in an aqueous solution of an antibody protein which is a monoclonal antibody wherein the antibody protein is an IgG immunoglobulin present at a concentration of 10 to 200 mg/mL during storage.

The term "high molecular weight species" as used herein, refers to any component of the antibody protein content which has an apparent molecular weight at least about double the molecular weight of the parent active antibody protein. That is, high molecular weight species are multimeric aggregates of the parent antibody protein. The multimeric aggregates may comprise the parent antibody protein molecules with considerably altered conformation or they may be an assembly of the parent protein units in the native or near-native conformation. The determination of high molecular weight species can be done using methods known in the art, including size exclusion chromatography, electrophoresis, analytical ultracentrifugation/sedimentation velocity, light scattering, dynamic light scattering, static light scattering and field flow fractionation.

The term "low molecular weight degradation products" as used herein, refers to any component of the antibody protein content which has an apparent molecular weight less than the molecular weight of the parent active antibody protein. That is, low molecular weight degradation products are fragments of the parent antibody protein. The determination of high molecular weight species can be done using methods known in the art, including size exclusion chromatography, electrophoresis, analytical ultracentrifugation/sedimentation velocity, light scattering, dynamic light scattering, static light scattering and field flow fractionation.

The term "related species" as used herein, refers to any component of the antibody protein content formed by a chemical modification of the parent antibody protein, such as deamidated species or oxidised species. Related species are suitably detected by cation-exchange chromatography, reversed-phase chromatography or capillary electrophoresis.

Suitably an aqueous solution of the invention is sufficiently stable such that it remains substantially free of visible particles after storage at 30 °C for at least one, two or three months. Visible particles are suitably detected using the 2.9.20. European Pharmacepoeia Monograph (Particulate Contamination: Visible Particles).

Suitably the aqueous solution of the invention is sufficiently stable such that the concentration of related species remains low upon extended storage.

In one embodiment, the aqueous solution of the invention retains at least 95%, e.g. at least 96%, e.g. at least 97%, e.g. at least 98%, e.g. at least 99% parent antibody protein (by weight of total antibody protein) after storage at 30°C for one, two or three months. The percentage of antibody protein (by weight of total antibody protein) may be determined by size-exclusion chromatography, cation-exchange chromatography, reversed-phase chromatography or capillary electrophoresis.

In one embodiment, the presence of the mixture of chelating agent which EDTA, and a C3 polyol which is selected from glycerol and 1,2-propanediol limits the increase in high molecular weight antibody protein species to no more than 5% (by weight of total antibody protein) after storage at 40°C for one month, suitably to no more than 3% and more suitably to no more than 2%. In one embodiment, the presence of a mixture of a chelating agent which EDTA, and a C3 polyol which is selected from glycerol and 1,2-propanediol limits the increase in high molecular weight antibody protein species to no more than 5% (by weight of total antibody protein) after storage at 2-8°C for up to two years, suitably to no more than 3% and more suitably to no more than 2%. Quantitation of high molecular weight species is as percent by weight of the total antibody protein (i.e. monoclonal antibody protein) in the aqueous solution.

In one embodiment, the presence of the mixture of a chelating agent which is EDTA, and a C3 polyol which is selected from glycerol and 1,2-propanediol limits the increase in high molecular weight antibody protein species by at least 10%, preferably by at least 25%, and more preferably by at least 50% compared with an aqueous solution lacking the chelating agent which is EDTA, and the C3 polyol which is selected from glycerol and 1,2-propanediol but otherwise identical, following storage under the same conditions and length of time.

In one embodiment, the presence of the mixture of a chelating agent which is EDTA, and a C3 polyol which is selected from glycerol and 1,2-propanediol maintains an aqueous solution of an antibody protein (i.e. monoclonal antibody protein) free of visible aggregates while formation of visible aggregates is observed in an aqueous solution lacking the mixture of the chelating agent which is EDTA, and a C3 polyol which is selected from glycerol and 1,2-propanediol but otherwise identical, following storage under the same conditions and for the same length of time. Quantification of visible aggregates can be performed by turbidity or other types of light scattering measurement.

Suitably, the aqueous solution of the invention comprises no more than 5% (by weight of total protein) high molecular weight species after storage at 40°C for at least one, two or three months. In one embodiment, the amount of high molecular weight species increases by no more than 5% (by weight of total antibody protein), preferably no more than 3%, after storage at 40°C for at least one, two or three months. Quantitation of high molecular weight species is as percent by weight of the total antibody protein in the aqueous solution.

Suitably, the aqueous solution of the invention should exhibit an increase in high molecular weight species during storage which is at least 10% lower, preferably at least 25% lower, more preferably at least 50% lower, than an aqueous solution lacking a mixture of a chelating agent which is EDTA, and a C3 polyol which is selected from glycerol and 1,2-propanediol but otherwise identical, following storage under the same conditions and length of time.

In one embodiment, the aqueous solution of the invention is a pharmaceutical composition suitable for administration of a therapeutic antibody protein to a subject in need thereof. Such compositions can be used in a method for administering the therapeutic protein to the subject.

Also described is a method for administering a therapeutic antibody protein to a subject in need thereof. The method comprises the step of administering an aqueous solution comprising an antibody protein, a chelating agent which is a multi-anion, and a C3 polyol. Preferably the composition is administered by intravenous, subcutaneous or intramuscular injection, or infusion. More preferably the composition is administered by subcutaneous injection.

Also described is a packaged pharmaceutical composition suitable for administration to a subject in need thereof. The pharmaceutical composition comprises an aqueous solution comprising an antibody protein, a chelating agent which is a multi-anion, and a C3 polyol. The pharmaceutical composition is preferably packaged in a vial suitable for introduction of a needle for removal of the solution. In one embodiment, the pharmaceutical composition is packaged in a glass vial with a rubber stopper. The packaged pharmaceutical composition can be provided as a kit, further comprising instructions for use and, optionally, a syringe suitable for intramuscular or subcutaneous administration. Alternatively, the packaged pharmaceutical composition can be provided in the form of a pre-filled disposable syringe suitable for intramuscular or subcutaneous administration. A pre-filled auto-injector device would also be suitable for intramuscular or subcutaneous administration.

The term "pharmaceutically acceptable", as used herein, refers to components of a pharmaceutical composition which are suitable for the intended use and mode of administration to the body of a human or an animal, such as a mammal, without undue adverse consequences, such as toxicity, irritation, and allergic response and with a reasonable risk/benefit ratio.

### ABBREVIATIONS

- EDTA: ethylenediaminetetraacetate
- PEG: polyethylene glycol
- HMWS: high molecular weight species
- SEC: size exclusion chromatography
- CEX: cation-exchange chromatography

### EXAMPLES

### Materials

EDTA disodium salt (Mw 372 Da), 1,2-propanediol (Mw 76 Da), glycerol (Mw 92 Da), mannitol (Mw 182 Da), NaCl (Mw 58 Da), trehalose (Mw 342 Da) were obtained from Sigma Aldrich.

### Methods of assessing stability of an antibody protein

### (a) Visual assessment

Visible particles are suitably detected using the 2.9.20. European Pharmacepoeia Monograph (Particulate Contamination: Visible Particles). The apparatus required consists of a viewing station comprising:
- a matt black panel of appropriate size held in a vertical position
- a non-glare white panel of appropriate size held in a vertical position next to the black panel
- an adjustable lampholder fitted with a suitable, shaded, white-light source and with a suitable light diffuser (a viewing illuminator containing two 13 W fluorescent tubes, each 525 mm in length, is suitable). The intensity of illumination at the viewing point is maintained between 2000 lux and 3750 lux.

Any adherent labels are removed from the container and the outside washed and dried. The container is gently swirled or inverted, ensuring that air bubbles are not introduced, and observed for about 5 s in front of the white panel. The procedure is repeated in front of the black panel. The presence of any particles is recorded.

The visual scores are ranked as follows:
Visual score 1: Clear solution, virtually free of particles
Visual score 2: ~ 5 very small particles
Visual score 3: ~10-20 very small particles
Visual score 4: 20-50 particles, including larger particles
Visual score 5: >50 particles, including larger particles

Whilst the particles in samples with visual scores 4 and 5 are clearly detectable on casual visual assessment under normal light, samples with visual score 1-3 generally appear as clear solutions on the same assessment. Samples with visual scores 1-3 are considered to be "Pass"; samples with visual score 4-5 are considered to be "Fail".

### (b) Size exclusion chromatography (SEC)

The amount of high molecular weight species is measured using a 300×7.8 mm S3000 (or equivalent) size-exclusion column with a guard column. The mobile phase is potassium phosphate pH 6.5, with a flow rate of 0.4 ml/min, injection volume of 1 µl and detected at 210 and 280 nm. The results are expressed as % high molecular species (HMWS), i.e. sum of all peak areas corresponding to aggregated protein over the sum of all protein-related peaks on the chromatogram. A small time-point to time-point variability can be observed in terms of absolute values of %HMWS, for example due to repeated size-exclusion column use. However, within a given time-point the samples are tested using the column in the same condition, so the values generated within the time-point represent a very good indication of the relative stability of the protein in the aqueous solutions tested.

### (b) Cation-exchange chromatography (CEX)

The amount of related species is measured using a Protein-Pak Hi Res SP column. Mobile phase A is 20 mM sodium phosphate (pH 6.5); mobile phase B is 20 mM sodium phosphate + 0.5 M NaCl (pH 6.0). The following gradient elution is used: 0 min - 100% A, 4 min - 80% A, 10 min - 55% A, 12 min - 0% A. Flow rate of 1.0 ml/min; injection volume is 3 µl, with UV detection at 214 nm. The results are expressed as % main peak (i.e. native protein), % acidic species and % basic species. % Related species = % acidic species + % basic species.

### Reference Example 1

The effect of EDTA and C3 polyols on the stability of abatacept (125 mg/ml) was investigated. The effect was tested in a background solution containing sodium phosphate (5 mM) and polysorbate 80 (0.5 mg/ml). All formulations tested were adjusted to pH 6.5. Additional excipients in the formulations tested are shown in Table 1.

**Table 1: Additional components in formulations of abatacept tested. All formulations contained abatacept (125 mg/ml), sodium phosphate (5 mM) and polysorbate 80 (0.5 mg/ml) and were adjusted to pH 6.5.**

| | NaCl (mM) | Trehalose (mM) | 1,2-propanediol (mM) | EDTA(mM) |
|---|---|---|---|---|
| Formulation 1 | 150 | 0 | 0 | 0 |
| Formulation 2 | 0 | 300 | 0 | 0 |
| Formulation 3 | 0 | 0 | 300 | 0 |
| Formulation 4 | 150 | 0 | 0 | 1 |
| Formulation 5 | 150 | 0 | 0 | 10 |
| Formulation 6 | 150 | 0 | 0 | 50 |
| Formulation 7 | 0 | 300 | 0 | 1 |
| Formulation 8 | 0 | 300 | 0 | 10 |
| Formulation 9 | 0 | 300 | 0 | 50 |
| Formulation 10 | 0 | 0 | 300 | 1 |
| Formulation 11 | 0 | 0 | 300 | 10 |
| Formulation 12 | 0 | 0 | 300 | 50 |

Stability of Formulations 1-12 (Table 1) was tested at 25 °C and 40 °C by visual assessment and size-exclusion chromatography (SEC). Results are shown in Tables 2 and 3. It was shown that in the absence of EDTA the stability of abatacept was slightly better in the presence of an uncharged tonicity modifier (trehalose or 1,2-propanediol) than in the presence of a charged tonicity modifier (NaCl). Addition of EDTA appeared to improve the stability of abatacept, both with respect to visual assessment and with respect to the formation of HMWS. The degree of improvement was greater in compositions comprising the uncharged species. Surprisingly, the degree of improvement was greater in compositions comprising a C3 polyol (1,2-propanediol) than in compositions comprising a larger polyol (trehalose). This indicates synergistic effect between EDTA and 1,2-propanediol. Whilst the highest level of EDTA tested (50 mM) resulted in the best stability with respect to high molecular weight species it also appeared to result in slightly worse visual score. This could be due to the fact that more soluble aggregates (i.e. HMWS) are converted to insoluble aggregates in the presence of high concentration of EDTA.

**Table 2: Visual scores of abatacept Formulations 1-12 following storage at 25 °C and 40 °C. Visual score 1: clear solution, virtually free of particles; visual score 2: ~ 5 very small particles; visual score 3: ~10-20 very small particles; visual score 4: 20-50 particles, including larger particles; visual score 5: >50 particles, including larger particles**

| | T = 0 weeks | T = 10 weeks (25°C) | T = 10 weeks (40°C) |
|---|---|---|---|
| Formulation 1 | 1 | 4 | 5 |
| Formulation 2 | 1 | 3 | 4 |
| Formulation 3 | 1 | 3 | 4 |
| Formulation 4 | 1 | 2 | 4 |
| Formulation 5 | 1 | 2 | 5 |
| Formulation 6 | 1 | 3 | 5 |
| Formulation 7 | 1 | 2 | 3 |
| Formulation 8 | 1 | 2 | 3 |
| Formulation 9 | 1 | 3 | 3 |
| Formulation 10 | 1 | 1 | 2 |
| Formulation 11 | 1 | 1 | 1 |
| Formulation 12 | 1 | 1 | 2 |

**Table 3: Stability of abatacept (125 mg/ml) in Formulations 1-12 assessed by SEC. Formation of HMWS was assessed following storage at 25°C and 40°C.**

| | HMWS (%) | | |
|---|---|---|---|
| | T = 0 weeks | T = 10 weeks (25°C) | T = 10 weeks (40°C) |
| Formulation 1 | 0.61 | 3.58 | 14.73 |
| Formulation 2 | 0.60 | 3.21 | 12.72 |
| Formulation 3 | 0.55 | 3.49 | 13.15 |
| Formulation 4 | 0.59 | 3.18 | 13.78 |
| Formulation 5 | 0.60 | 3.18 | 13.26 |
| Formulation 6 | 0.54 | 3.06 | 13.00 |
| Formulation 7 | 0.62 | 2.79 | 10.26 |
| Formulation 8 | 0.58 | 2.83 | 10.44 |
| Formulation 9 | 0.61 | 2.46 | 9.99 |
| Formulation 10 | 0.60 | 1.99 | 8.97 |
| Formulation 11 | 0.60 | 1.98 | 8.29 |
| Formulation 12 | 0.57 | 2.10 | 8.13 |

### Example 2

The effect of EDTA and polyols on stability of bevacizumab (25 mg/ml) was investigated at 40 °C. The effect was tested in a background solution containing sodium phosphate (5 mM) and polysorbate 20 (0.4 mg/ml). All formulations tested were adjusted to pH 6.2. Additional excipients in the formulations tested are shown in Table 4. The stability was also compared to the composition of the currently marketed bevacizumab product (Avastin^{®}).

**Table 4: Additional components in formulations of abatacept tested. All formulations contained bevacizumab (25 mg/ml) and polysorbate 20 (0.5 mg/ml) and were adjusted to pH 6.2.**

| | Sodium phosphate (mM) | NaCl (mM) | Trehalose (mM) | Mannitol (mM) | Glycerol (mM) | EDTA (mM) |
|---|---|---|---|---|---|---|
| Formulation 1 = Composition of Avastin^{®} | 50.4 | | 158 | | | |
| Formulation 2 | 5 | 130 | | | | |
| Formulation 3 | 5 | | 300 | | | |
| Formulation 4 | 5 | | | 300 | | |
| Formulation 5 | 5 | | | | 300 | |
| Formulation 6 | 5 | 130 | | | | 10 |
| Formulation 7 | 5 | 130 | | | | 50 |
| Formulation 8 | 5 | | | | 300 | 10 |
| Formulation 9 | 5 | | | | 300 | 50 |

Stability of Formulations 1-9 (Table 4) was tested at 40 °C by visual assessment. Results are shown in Table 5. Formulation 1 (i.e. the composition of Avastin^{®}) resulted in visual score 5 following 10 weeks storage at 40 °C. Similarly, visual score 5 was reached in compositions containing 5 mM sodium phosphate and either charged tonicity modifier (NaCl) or an uncharged tonicity modifier (mannitol, trehalose or glycerol). Better visual score was observed in the presence of EDTA (10 or 50 mM). However, the use of EDTA in the presence of NaCl still resulted in worse visual scores than the use of EDTA in the presence of a C3 polyol (glycerol), indicating a synergistic effect between EDTA and the C3 polyol.

**Table 5: Visual scores of bevacizumab Formulations 1-9 following storage at 40 °C. Visual score 1: clear solution, virtually free of particles; visual score 2: ~ 5 very small particles; visual score 3: ~10-20 very small particles; visual score 4: 20-50 particles, including larger particles; visual score 5: >50 particles, including larger particles.**

| | T = 0 weeks | T = 10 weeks (40°C) |
|---|---|---|
| Formulation 1 | 1 | 5 |
| Formulation 2 | 1 | 5 |
| Formulation 3 | 1 | 5 |
| Formulation 4 | 1 | 5 |
| Formulation 5 | 1 | 5 |
| Formulation 6 | 1 | 3 |
| Formulation 7 | 1 | 3 |
| Formulation 8 | 1 | 1 |
| Formulation 9 | 1 | 2 |

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

## Claims

1. An aqueous solution comprising an antibody protein which is a monoclonal antibody wherein the antibody protein is an IgG immunoglobulin present at a concentration of 10 to 200 mg/ml and a stabilizing mixture of (i) a chelating agent which is EDTA present at a concentration of 0.1 to 10 mM; (ii) a C3 polyol which is selected from glycerol and 1,2-propanediol present at a concentration of 100 to 500 mM; (iii) a non-ionic surfactant present at a concentration of 10 to 2000 µg/mL and (iv) a buffer.

2. Use of a mixture of (i) a chelating agent which is EDTA present at a concentration of 0.1 to 10 mM; (ii) a C3 polyol which is selected from glycerol and 1,2-propanediol present at a concentration of 100 to 500 mM; (iii) a non-ionic surfactant present at a concentration of 10 to 2000 µg/ml; and (iv) a buffer, for stabilizing an antibody protein which is a monoclonal antibody wherein the antibody protein is an IgG immunoglobulin present at a concentration of 10 to 200 mg/ml in an aqueous solution to storage.

3. The aqueous solution or use of claim 1 or claim 2, wherein the C3 polyol is 1,2-propanediol.

4. The aqueous solution or use of claim 1 or claim 2, wherein the C3 polyol is glycerol.

5. The aqueous solution or use of claim 1 or claim 2, wherein the C3 polyol is a mixture of 1,2-propanediol and glycerol.

6. The aqueous solution or use of any one of claims 1 to 5, wherein the monoclonal antibody is a murine antibody, a chimeric antibody, a humanized antibody or a human antibody.

7. The aqueous solution or use of any one of claims 1 to 6, wherein the monoclonal antibody is selected from trastuzumab, rituximab, bevacizumab, cetuximab and ipilimumab.

8. The aqueous solution or use of claim 7, wherein the monoclonal antibody is bevacizumab.

9. The aqueous solution or use of any one of claims 1 to 8, wherein the pH of the solution is between pH 4.0 and pH 8.0.

10. The aqueous solution or use of any one of claims 1 to 9, wherein the buffer is selected from the group consisting of histidine, succinate, maleate, acetate, phosphate and TRIS.

11. The aqueous solution or use of any one of claims 1 to 10, wherein the non-ionic surfactant is selected from:
an alkyl glycoside;
a polysorbate surfactant;
an alkyl ether of polyethylene glycol;
a block copolymer of polyethylene glycol and polypropylene glycol; and
an alkylphenyl ether of polyethylene glycol.

12. The aqueous solution or use of claim 11, wherein the non-ionic surfactant is a polysorbate surfactant.

13. The aqueous solution or use of any one of claims 1 to 12, further comprising an uncharged tonicity modifier.

14. The aqueous solution or use of any one of claims 1 to 13, further comprising a charged tonicity modifier.

15. The aqueous solution or use of any of claims 1 to 14, further comprising a preservative selected from the group consisting of phenol, m-cresol, chlorocresol, benzyl alcohol, propylparaben, methylparaben, benzalkonium chloride and benzethonium chloride.

## Patentansprüche

1. Wässrige Lösung, umfassend ein Antikörperprotein, das ein monoklonaler Antikörper ist, wobei das Antikörperprotein ein IgG-Immunglobulin ist, das in einer Konzentration von 10 bis 200 mg/ml vorliegt, und eine stabilisierende Mischung aus (i) einem Chelatbildner, der EDTA ist, in einer Konzentration von 0,1 bis 10 mM vorliegt; (ii) einem C3-Polyol, das ausgewählt ist aus Glyzerin und 1,2-Propandiol, das in einer Konzentration von 100 bis 500 mM vorliegt; (iii) ein nichtionisches Tensid, das in einer Konzentration von 10 bis 2000 µg/ml vorliegt und (iv) einen Puffer.

2. Verwendung einer Mischung aus (i) einem Chelatbildner, der EDTA ist, in einer Konzentration von 0,1 bis 10 mM; (ii) einem C3-Polyol, das aus Glycerin und 1,2-Propandiol ausgewählt ist, in einer Konzentration von 100 bis 500 mM; (iii) einem nicht ionisches Tensid, das in einer Konzentration von 10 bis 2000 µg/ml vorhanden ist; und (iv) einen Puffer, zur Stabilisierung eines Antikörperproteins, das ein monoklonaler Antikörper ist, wobei das Antikörperprotein ein IgG-Immunglobulin ist, das in einer Konzentration von 10 bis 200 mg/ml in einer wässrigen Lösung zur Lagerung vorhanden ist.

3. Wässrige Lösung oder Verwendung nach Anspruch 1 oder Anspruch 2, wobei das C3-Polyol 1,2-Propandiol ist.

4. Wässrige Lösung oder Verwendung nach Anspruch 1 oder Anspruch 2, wobei das C3-Polyol Glycerin ist.

5. Wässrige Lösung oder Verwendung nach Anspruch 1 oder Anspruch 2, wobei das C3-Polyol 1,2-Propandiol ist.

6. Wässrige Lösung oder Verwendung nach einem der Ansprüche 1 bis 5, wobei der monoklonale Antikörper ein muriner Antikörper, ein chimärer Antikörper, ein humanisierter Antikörper oder ein menschlicher Antikörper ist.

7. Wässrige Lösung oder Verwendung nach einem der Ansprüche 1 bis 6, wobei der monoklonale Antikörper ausgewählt ist aus Trastuzumab, Rituximab, Bevacizumab, Cetuximab und Ipilimumab.

8. Wässrige Lösung oder Verwendung nach Anspruch 7, wobei der monoklonale Antikörper Bevacizumab ist.

9. Wässrige Lösung oder Verwendung nach einem der Ansprüche 1 bis 8, wobei der pH-Wert der Lösung zwischen pH 4,0 und pH 8,0 liegt.

10. Wässrige Lösung oder Verwendung nach einem der Ansprüche 1 bis 9, wobei der Puffer ausgewählt ist aus der Gruppe bestehend aus Histidin, Succinat, Maleat, Azetat, Phosphat und TRIS.

11. Wässrige Lösung oder Verwendung nach einem der Ansprüche 1 bis 10, wobei das nichtionische Tensid ausgewählt ist aus:
ein Alkylglycosid;
ein Polysorbat-Tensid;
ein Alkylether aus Polyethylenglykol;
ein Blockcopolymer aus Polyethylenglykol und Polypropylenglykol; und
ein Alkylether aus Polyethylenglykol.

12. Wässrige Lösung oder Verwendung nach Anspruch 11, wobei das nichtionische Tensid ein Polysorbat-Tensid ist.

13. Wässrige Lösung oder Verwendung nach einem der Ansprüche 1 bis 12, ferner umfassend ein ungeladenes Tonizitätsmodifizierungsmittel.

14. Wässrige Lösung oder Verwendung nach einem der Ansprüche 1 bis 13, ferner umfassend ein geladenes Tonizitätsmodifizierungsmittel.

15. Wässrige Lösung oder Verwendung nach einem der Ansprüche 1 bis 14, ferner umfassend ein Konservierungsmittel, das geeigneterweise ausgewählt ist aus der Gruppe, bestehend aus Phenol, m-Cresol, Chlorcresol, Benzylalkohol, Propylparaben, Methylparaben, Benzalkoniumchlorid und Benzethoniumchlorid.

## Revendications

1. Solution aqueuse comprenant une protéine d'anticorps qui est un anticorps monoclonal dans laquelle la protéine d'anticorps est une immunoglobuline IgG présente à une concentration de 10 à 200 mg/ml et un mélange stabilisant (i) d'un agent chélatant qui est EDTA présent à une concentration de 0,1 à 10 mM ; (ii) d'un polyol C3 qui est choisi parmi le glycérol et le 1,2-propanediol présent à une concentration de 100 à 500 mM ; (iii) d'un tensioactif non ionique présent à une concentration de 10 à 2 000 µg/ml et (iv) d'un tampon.

2. Utilisation d'un mélange (i) d'un agent chélatant qui est EDTA présent à une concentration de 0,1 à 10 mM ; (ii) d'un polyol C3 qui est choisi parmi le glycérol et le 1,2-propanediol présent à une concentration de 100 à 500 mM ; (iii) d'un tensioactif non ionique présent à une concentration de 10 à 2 000 µg/ml ; et (iv) d'un tampon, pour stabiliser une protéine d'anticorps qui est un anticorps monoclonal dans laquelle la protéine d'anticorps est une immunoglobuline IgG présente à une concentration de 10 à 200 mg/ml dans une solution aqueuse pour stockage.

3. Solution aqueuse ou utilisation selon la revendication 1 ou la revendication 2, dans laquelle le polyol C3 est le 1,2-propanediol.

4. Solution aqueuse ou utilisation selon la revendication 1 ou la revendication 2, dans laquelle le polyol C3 est le glycérol.

5. Solution aqueuse ou utilisation selon la revendication 1 ou la revendication 2, dans laquelle le polyol C3 est le 1,2-propanediol et le glycérol.

6. Solution aqueuse ou utilisation selon l'une quelconque des revendications 1 à 5, dans laquelle l'anticorps monoclonal est un anticorps murin, un anticorps chimérique, un anticorps humanisé ou un anticorps humain.

7. Solution aqueuse ou utilisation selon l'une quelconque des revendications 1 à 6, dans laquelle l'anticorps monoclonal est choisi parmi le trastuzumab, le rituximab, le bévacizumab, le cétuximab et l'ipilimumab.

8. Solution aqueuse ou utilisation selon la revendication 7, dans laquelle l'anticorps monoclonal est le bévacizumab.

9. Solution aqueuse ou utilisation selon l'une quelconque des revendications 1 à 8, dans laquelle le pH de la solution est compris entre pH 4,0 et pH 8,0.

10. Solution aqueuse ou utilisation selon l'une quelconque des revendications 1 à 9, dans laquelle le tampon est choisi dans le groupe constitué de l'histidine, du succinate, du maléate, de l'acétate, du phosphate et du TRIS.

11. Solution aqueuse ou utilisation selon l'une quelconque des revendications 1 à 10, dans laquelle le tensioactif non ionique est choisi parmi :
un alkylglycoside ;
un tensioactif polysorbate ;
un éther alkylique de polyéthylène glycol ;
un copolymère séquencé de polyéthylène glycol et de polypropylène glycol ; et
un éther alkylphénylique de polyéthylène glycol .

12. Solution aqueuse ou utilisation selon la revendication 11, dans laquelle le tensioactif non ionique est un tensioactif polysorbate.

13. Solution aqueuse ou utilisation selon l'une quelconque des revendications 1 à 12, comprenant également un modificateur de tonicité non chargé.

14. Solution aqueuse ou utilisation selon l'une quelconque des revendications 1 à 13, comprenant également un modificateur de tonicité non chargé.

15. Solution aqueuse ou utilisation selon l'une quelconque des revendications 1 à 14, comprenant également un conservateur choisi dans le groupe constitué du phénol, du m-crésol, du chlorocrésol, de l'alcool benzylique, du propylparabène, du méthylparabène, du chlorure de benzalkonium et du chlorure de benzéthonium.
